# EUROPEAN PATENT SPECIFICATION

(11) **EP 3 091 958 B1**
(45) Date of publication and mention of the grant of the patent: **03.04.2024**
(21) Application number: 13814003.3
(22) Date of filing: 10.12.2013
(51) Int. Cl.: A61K 8/34, A61Q 5/00, A61Q 5/02, A61Q 5/12, A61K 8/31, A61K 8/37, A61K 8/41

(54) **COSMETIC COMPOSITION COMPRISING CATIONIC SURFACTANT(S), ANTIDANDRUFF AGENT(S), LIQUID NON-SILICEOUS FATTY SUBSTANCE(S), AND NON-POLYMERIC THICKENING AGENT(S)**
KOSMETISCHE ZUSAMMENSETZUNG MIT KATIONISCHEN TENSIDEN, ANTISCHUPPENMITTELN, SILIKONFREIEN FLÜSSIGEN FETTSUBSTANZEN UND NICHTPOLYMEREN VERDICKUNGSMITTELN
COMPOSITION COSMÉTIQUE COMPRENANT UN OU PLUSIEURS TENSIOACTIFS CATIONIQUES, AGENTS ANTIPELLICULAIRES, CORPS GRAS LIQUIDES NON SILICEUX ET AGENTS ÉPAISSISSANTS NON POLYMÈRES

(43) Date of publication of application: 16.11.2016
(73) Proprietor: L'OREAL, 75008 Paris (FR)
(72) Inventor: APRIGLIANO FERNANDES, Pedro, 21930-220 Rio de Janeiro - RJ (BR); PATARO, Marcele, 20520-052 Rio de Janeiro - RJ (BR)
(74) Representative: Casalonga
(86) International application number: PCT/BR2013/000550
(87) International publication number: WO 2015/085376

(56) References cited:
- JP-A- 58 032 813
- JP-A- 58 032 814
- DATABASE GNPD [Online] MINTEL; 1 February 2013 (2013-02-01), "Anti-Dandruf Conditioner", XP002726339, Database accession no. 1978343
- DATABASE GNPD [Online] MINTEL; 1 March 2013 (2013-03-01), "Purifying Anti-Dandruff Lotion", XP002726340, Database accession no. 2035435
- DATABASE GNPD [Online] MINTEL; 1 November 2001 (2001-11-01), "Nourishing Care", Database accession no. 120910
- DATABASE GNPD [Online] MINTEL; 1 September 2013 (2013-09-01), "Conditioner", Database accession no. 2286535
- DATABASE GNPD [Online] MINTEL; 8 October 2010 (2010-10-08), anonymous: "Conditioner", XP055564987, retrieved from www.gnpd.com Database accession no. 1412151
- DATABASE GNPD [Online] MINTEL; 21 May 2013 (2013-05-21), anonymous: "Sensitive Scalp Dandruff Shampoo", XP055617851, retrieved from www.gnpd.com Database accession no. 2074830
- DATABASE GNPD [Online] MINTEL; 2 July 2012 (2012-07-02), anonymous: "Treatment Conditioner", XP055710117, retrieved from www.gnpd.com Database accession no. 1830314
- Anonymous: "GNPD - Cocamide MEA", , 17 July 2020 (2020-07-17), XP55715922, Retrieved from the Internet: URL:https://www.gnpd.com/sinatra/ingredien ts/7112/ [retrieved on 2020-07-17]

## Description

The present invention relates to a cosmetic composition particularly for treating keratin materials, in particular human keratin fibers such as the hair, comprising one or more particular cationic surfactants, one or more particular antidandruff agent, one or more liquid non-siliceous fatty substances, one or more non-polymeric thickening agent and one or more aminosilicones, the one or more liquid non-siliceous fatty substances comprising isopropyl myristate.

Many cosmetic care products are known in the field of hair hygiene. For example, the purpose of the corresponding compositions is to impart good cosmetic properties to the hair.

Conventional care products for keratin materials and in particular for the hair and scalp, for example conditioners or masks, are usually in the form of more or less viscous creams.

In addition, haircare compositions, whether they are rinse-out or leave-in products, consist for the vast majority of water, surfactants and polymers. These starting materials do not always make it possible to incorporate other desired starting materials, or in the desired proportion. Specifically, such an incorporation may occasionally lead to destabilization such as flocculation or sedimentation of particles.

In particular, the incorporation of an antidandruff agent in order to combat the formation of dandruff may be detrimental to the stability of the composition, specifically to its stability over time, to the adjustment of the viscosity of the compositions and to the deposition of the conditioning agents that may be present in the compositions.

Within the meaning of the invention, the expression "being detrimental to the stability of the composition" means that the aspect of the composition evolves with time, for example with a strong modification of its viscosity, and/or with phase separation and/or crystallization and/or exudation.

Currently, there is a need for haircare products imparting good cosmetic properties such as disentangling, feel, softness, lightness and anti-frizz, to hair, and in particular to sensitized and voluminous hair for example Brazilian hair, while greatly limiting, even preventing the appearance of dandruffs.

The inventors have searched to meet said need and have discovered unexpectedly that the combination of one or more particular cationic surfactants, one or more particular antidandruff agents, one or more liquid non-siliceous fatty substances, one or more non-polymeric thickening agents and one or more aminosilicones, the one or more liquid non-siliceous fatty substances comprising isopropyl myristate, allows obtaining the searched effects and overcoming the above drawbacks.

In particular, this combination makes it possible to appreciably have good in-use conditions as well as good cosmetic properties as described above, while at the same time reducing the production of dandruff with good efficacy, and to do so in a long-lasting manner.

Therefore, a subject-matter of the present invention is directed to a cosmetic composition for treating keratin materials, in particular human keratin fibers such as the hair, comprising one or more cationic surfactants as defined below, one or more antidandruff agents as defined below, one or more liquid non-siliceous fatty substances, one or more non-polymeric thickening agents and one or more aminosilicones, the one or more liquid non-siliceous fatty substances comprising isopropyl myristate.

The composition of the invention may be a leave-on cosmetic composition that controls discipline and treats the sensitized and volumous hair with antidandruff efficacy.

In particular, the cosmetic composition allows to obtain excellent cosmetic properties such as disentangling, feel, softness, lightness and anti-frizz. Its texture is very pleasant.

Moreover, the composition of the invention remains stable over time at room temperature (25°C) and preferably at temperature range between 4°C and 45°C.

The present invention also relates to a cosmetic treatment process, preferably for conditioning keratin fibers, in particular the hair, which consists in applying to the keratin materials a composition according to the invention, and optionally in rinsing.

Another subject-matter is the composition of the invention for use in the treatment of dandruff.

Other subjects, characteristics, aspects and advantages of the invention will emerge even more clearly on reading the description and the example that follows.

In the text herein below, unless otherwise indicated, the limits of a range of values are included in that range, for example in the expressions "between" and "ranging from ... to ...".

Moreover, the expression "at least one" used in the present description is equivalent to the expression "one or more".

According to the invention, the cosmetic composition comprises:
- one or more cationic surfactants chosen from cetyltrimethylammonium, behenyltrimethylammonium, dipalmitoylethylhydroxyethylmethylammonium and distearoylethylhydroxyethylmethylammonium salts, and mixtures thereof,
- one or more antidandruff agents,
- one or more liquid non-siliceous fatty substances, and
- one or more non-polymeric thickening agents,
the one or more antidandruff agents being chosen from 1-hydroxy-2-pyridone derivatives represented by formula (V) as defined below, and the one or more liquid non-siliceous fatty substances comprising isopropyl myristate.

Among the cationic surfactants present in the composition according to the invention, it is more particularly preferred to choose behenyltrimethylammonium chloride, cetyltrimethylammonium chloride, dipalmitoylethylhydroxyethylammonium methosulfate and distearoylethylhydroxyethylmethylammonium methosulfate, and better still behenyltrimethylammonium chloride, cetyltrimethylammonium chloride, and mixtures thereof.

The cationic surfactant(s) are added in the composition according to the invention in an amount ranging from 0.05% to 10% by weight, more preferentially from 0.1% to 5% by weight, better still from 0.5% to 5% by weight and even better still from 0.5% to 2% by weight relative to the total weight of the composition.

As mentioned above, the composition according to the invention comprises one or more antidandruff agents.

The antidandruff agents that are used according to the invention are chosen from:
1-hydroxy-2-pyridone derivatives represented by formula (V): 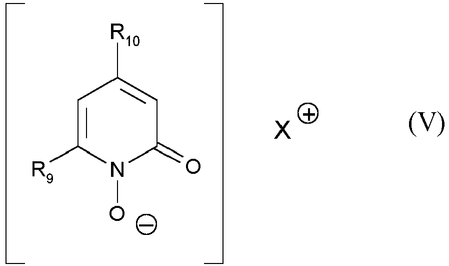
in which R₉ represents an alkyl group containing from 1 to 17 carbon atoms, an alkenyl group containing from 2 to 17 carbon atoms, a cycloalkyl group containing from 5 to 8 carbon atoms, a bicycloalkyl group containing from 7 to 9 carbon atoms; a cycloalkylalkyl group, an aryl group, an aralkyl group with an alkyl containing from 1 to 4 carbon atoms, an arylalkenyl group with an alkenyl containing from 2 to 4 carbon atoms, aryloxyalkyl or arylmercaptoalkyl with an alkyl containing from 1 to 4 carbon atoms, a furylalkenyl group with an alkenyl or a furyl containing from 2 to 4 carbon atoms, an alkoxy group containing from 1 to 4 carbon atoms, a nitro group, a cyano group or a halogen atom,
R₁₀ represents a hydrogen atom, a C₁-C₄ alkyl group, a C₂-C₄ alkenyl group, a halogen atom, a phenyl group or a benzyl group, and
X represents an organic base, an alkali metal or alkaline-earth metal ion or an ammonium ion.

Examples of compounds of formula (V) include derivatives of 1-hydroxy-4-methyl-2-pyridone, 1-hydroxy-6-methyl-2-pyridone, 1-hydroxy-4,6-dimethyl-2-pyridone, 1-hydroxy-4-methyl-6-(2,4,4-trimethylpentyl)-2-pyridone, 1-hydroxy-4-methyl-6-cyclohexyl-2-pyridone, 1-hydroxy-4-methyl-6-(methylcyclohexyl)-2-pyridone, 1-hydroxy-4-methyl-6-(2-bicyclo[2,2,1]heptyl)-2-pyridone, 1-hydroxy-4-methyl-6-(4-methylphenyl)-2-pyridone, 1-hydroxy-4-methyl-6-[1-(4-nitrophenoxy)butyl]-2-pyridone, 1-hydroxy-4-methyl-6-(4-cyanophenoxymethyl)-2-pyridone, 1-hydroxy-4-methyl-6-(phenylsulfonylmethyl)-2-pyridone and 1-hydroxy-4-methyl-6-(4-bromobenzyl)-2-pyridone.

The compounds of formula (V) may be used in the form of salts with organic or mineral bases.

Examples of organic bases are especially alkanolamines of low molecular weight such as ethanolamine, diethanolamine, N-ethylethanolamine, triethanolamine, diethylaminoethanol and 2-amino-2-methylpropanediol; non-volatile bases such as ethylenediamine, hexamethylenediamine, cyclohexylamine, benzylamine and N-methylpiperazine; quaternary ammonium hydroxides, such as trimethylbenzylammonium hydroxide; guanidine and derivatives thereof, and particularly alkyl derivatives thereof. Examples of mineral bases are especially salts of alkali metals, e.g. sodium or potassium; ammonium salts, salts of alkaline-earth metals, such as magnesium and calcium; salts of di-, tri- or tetravalent cationic metals, e.g. zinc, aluminium or zirconium.

Alkanolamines, ethylenediamine and mineral bases such as alkali metal salts are preferred.

The 1-hydroxy-4-methyl-6-(2,4,4-trimethylpentyl)-2-pyridone salts thereof are particularly preferred.

A compound of formula (V) that is particularly preferred is the one for which:
R₉ denotes the group 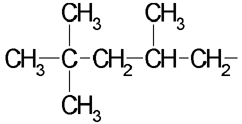
R₁₀ denotes a methyl group, and
X⁺ denotes N⁺H₃CH₂CH₂OH.

This compound is sold, for example, under the name Octopirox (1-hydroxy-4-methyl-6-(2,4,4-trimethylpentyl)-2-pyridone, monoethanolamine salt) by the company Hoechst.

The antidandruff agent(s) are added in the composition according to the invention in an amount preferentially ranging from 0.001% to 10% by weight, and more preferentially from 0.05% to 5% by weight, more preferably from 0.1 to 1% by weight, relative to the total weight of the composition.

The composition according to the invention contains one or more liquid non-siliceous fatty substances which comprise isopropyl myristate.

The term "fatty substance" means an organic compound that is insoluble in water at standard temperature (25°C) and at atmospheric pressure (760 mmHg, i.e. 1.013×10⁵ Pa), i.e. with a solubility of less than 5%, preferably of less than 1% and even more preferably of less than 0.1%. The non-siliceous fatty substances generally have in their structure a hydrocarbon-based chain comprising at least 6 carbon atoms and not comprising any siloxane groups. In addition, the fatty substances are generally soluble in organic solvents under the same temperature and pressure conditions, for instance chloroform, ethanol, benzene, liquid petroleum jelly or decamethylcyclopentasiloxane.

The term "non-siliceous fatty substance" means a fatty substance whose structure does not comprise any silicon atoms.

The fatty substances that may be used in the composition according to the invention are not oxyalkylenated and preferably do not contain any carboxylic acid COOH functions.

They are liquid at room temperature and at atmospheric pressure.

The liquid fatty substances of the invention preferably have a viscosity of less than or equal to 2 Pa.s, better still less than or equal to 1 Pa.s and even better still less than or equal to 0.1 Pa.s at a temperature of 25°C and at a shear rate of 1 s⁻¹.

Preferably, the liquid non-siliceous fatty substances used in the invention are chosen from liquid hydrocarbons, liquid fatty alcohols, liquid fatty esters, liquid fatty ethers and mixtures thereof.

Even more preferentially, they are chosen from liquid hydrocarbons, liquid fatty esters and mixtures thereof.

The term "liquid hydrocarbon" means a hydrocarbon composed solely of carbon and hydrogen atoms, which is liquid at standard temperature (25°C) and at atmospheric pressure (760 mmHg, i.e. 1.013 × 10⁵ Pa), which is especially of mineral or plant origin, preferably of plant origin.

More particularly, the liquid hydrocarbons are chosen from:
- linear or branched, optionally cyclic, C₆-C₁₆ alkanes. Examples that may be mentioned include hexane, undecane, dodecane, tridecane, and isoparaffins, for instance isohexadecane, isododecane and isodecane,
- linear or branched hydrocarbons of mineral, animal or synthetic origin with more than 16 carbon atoms, such as mineral oils, volatile or non-volatile liquid paraffins, petroleum jelly, liquid petroleum jelly, polydecenes, hydrogenated polyisobutene such as the product sold under the brand name Parleam^{®} by the company NOF Corporation, and squalane.

In one preferred variant, the liquid hydrocarbon(s) are chosen from mineral oils.

Mineral Oil is a liquid mixture of hydrocarbons obtained from petroleum. In the United States, Mineral Oil may be used as an active ingredient in OTC drug products. When used as an active drug ingredient, the established name is Mineral Oil. The labeling name in the EU will be, Paraffinum Liquidum, when regulations for ingredient labeling under the 6th Amendment to the EC Cosmetics Directive go into effect. It is known by CAS Nos. 8012-95-1 and 8042-47-5 and is available in numerous grades and under numerous technical names including heavy mineral oil, light mineral oil, liquid paraffin, liquid petrolatum, vaseline oil and paraffin oil. A non-comprehensive list of mineral oils and suppliers can be found in the International Cosmetic Ingredient Dictionary and Handbook, Seventh Edition, 1997, Edited by J. Wenninger and G.N. McEwen, Jr. Any type or grade of mineral oil may be used in accordance with the present invention. Blends of various grades and viscosities of mineral oils are also contemplated.

The term "liquid fatty alcohol" means a non-glycerolated and non-oxyalkylenated fatty alcohol, which is liquid at standard temperature (25°C) and at atmospheric pressure (760 mmHg, i.e. 1.013 × 10⁵ Pa).

Preferably, the liquid fatty alcohols of the invention comprise from 8 to 30 carbon atoms, more preferably from 10 to 24 carbon atoms. In particularly, they are mono-alcohols.

The liquid fatty alcohols of the invention may be saturated or unsaturated.

The saturated liquid fatty alcohols are preferably branched. They may optionally comprise in their structure at least one aromatic or non-aromatic ring. They are preferably acyclic.

More particularly, the liquid saturated fatty alcohols of the invention are chosen from octyldodecanol, stearyl alcohol, cetyl alcohol, isostearyl alcohol and 2-hexyldecanol, and their mixture.

Octyldodecanol is most particularly preferred.

The unsaturated liquid fatty alcohols contain in their structure at least one double or triple bond, and preferably one or more double bonds. When several double bonds are present, there are preferably 2 or 3 of them, and they may be conjugated or unconjugated.

These unsaturated fatty alcohols may be linear or branched.

They may optionally comprise in their structure at least one aromatic or non-aromatic ring. They are preferably acyclic.

More particularly, the unsaturated liquid fatty alcohols of the invention are chosen from oleyl alcohol, linoleyl alcohol, linolenyl alcohol and undecylenyl alcohol.

Oleyl alcohol is most particularly preferred.

The term "liquid fatty esters" means an ester derived from a fatty acid and/or from a fatty alcohol that is liquid at standard temperature (25°C) and at atmospheric pressure (760 mmHg, i.e. 1.013 × 10⁵ Pa).

The esters are preferably liquid esters of saturated or unsaturated, linear or branched C₁-C₂₆ aliphatic monoacids or polyacids and of saturated or unsaturated, linear or branched C₁-C₂₆ aliphatic monoalcohols or polyalcohols, the total number of carbon atoms of the esters being greater than or equal to 10.

Preferably, for the esters of monoalcohols, at least one from among the alcohol and the acid from which the esters of the invention are derived is branched.

Among the monoesters of monoacids and of monoalcohols, mention may be made of ethyl palmitate, isopropyl palmitate, alkyl myristates such as isopropyl myristate or ethyl myristate, isocetyl stearate, 2-ethylhexyl isononanoate, isononyl isononanoate, isodecyl neopentanoate and isostearyl neopentanoate.

Esters of C₄-C₂₂ dicarboxylic or tricarboxylic acids and of C₁-C₂₂ alcohols and esters of monocarboxylic, dicarboxylic or tricarboxylic acids and of C₄-C₂₆ dihydroxy, trihydroxy, tetrahydroxy or pentahydroxy non-sugar alcohols may also be used.

Mention may be made especially of: diethyl sebacate; diisopropyl sebacate; bis(2-ethylhexyl) sebacate; diisopropyl adipate; di-n-propyl adipate; dioctyl adipate; bis(2-ethylhexyl) adipate; diisostearyl adipate; bis(2-ethylhexyl) maleate; triisopropyl citrate; triisocetyl citrate; triisostearyl citrate; glyceryl trilactate; glyceryl trioctanoate; trioctyldodecyl citrate; trioleyl citrate; neopentyl glycol diheptanoate; diethylene glycol diisononanoate.

The composition may also comprise, as liquid fatty ester, sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides or polysaccharides.

Examples of suitable sugars that may be mentioned include sucrose, glucose, galactose, ribose, fucose, maltose, fructose, mannose, arabinose, xylose and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may comprise one to three conjugated or unconjugated carbon-carbon double bonds.

The esters according to this variant may also be chosen from mono-, di-, tri- and tetraesters, and polyesters, and mixtures thereof.

These esters may be chosen, for example, from oleates, laurates, palmitates, myristates, behenates, cocoates, stearates, linoleates, linolenates, caprates and arachidonates, or mixtures thereof, such as, in particular, oleopalmitate, oleostearate or palmitostearate mixed esters.

More particularly, use is made of monoesters and diesters and in particular of sucrose, glucose or methylglucose mono- or dioleates, stearates, behenates, oleopalmitates, linoleates, linolenates or oleostearates.

An example that may be mentioned is the product sold under the name Glucate^{®} DO by the company Amerchol, which is a methylglucose dioleate.

Finally, use may also be made of natural or synthetic glycerol esters of mono-, di- or triacids.

Among these, mention may be made of plant oils.

As oils of plant origin or synthetic triglycerides that may be used in the composition of the invention as liquid fatty esters, examples that may be mentioned include:
- triglyceride oils of plant or synthetic origin, such as liquid triglycerides of fatty acids containing from 6 to 30 carbon atoms, for instance heptanoic or octanoic acid triglycerides, or alternatively, for example, sunflower oil, corn oil, soybean oil, marrow oil, grapeseed oil, sesame seed oil, hazelnut oil, apricot oil, macadamia oil, arara oil, castor oil, avocado oil, olive oil, rapeseed oil, coconut oil, wheatgerm oil, sweet almond oil, apricot oil, safflower oil, candlenut oil, camellina oil, tamanu oil, babassu oil and pracaxi oil, caprylic/capric acid triglycerides, for instance those sold by the company Stéarineries Dubois or those sold under the names Miglyol^{®} 810, 812 and 818 by the company Dynamit Nobel, jojoba oil and shea butter oil.

Preferably, the esters are liquid esters of saturated linear or branched C₁-C₂₆, preferably C₄-C₂₂, more preferably C₆-C₂₀, aliphatic monoacids and of saturated linear or branched C₁-C₂₆, preferably C₂-C₂₂, more preferably C₂-C₂₀, aliphatic monoalcohols, the total number of carbon atoms of the esters being greater than or equal to 10; most preferably, at least one from among the alcohol and the acid from which the esters of the invention are derived being branched.

Liquid fatty esters derived from monoalcohols will preferably be used as esters according to the invention.

Isopropyl myristate and isopropyl palmitate are particularly preferred.

The liquid fatty ethers are chosen from liquid dialkyl ethers such as dicaprylyl ether.

In one preferred embodiment, the liquid non-siliceous fatty substance(s) is(are) selected from liquid hydrocarbons, for example mineral oil, liquid fatty esters such as isopropyl myristate and isopropyl palmitate, and mixtures thereof.

The liquid non-siliceous fatty substance(s) are present in the composition according to the invention in an amount preferentially ranging from 0.1% to 30% by weight, and more preferentially from 0.5% to 20% by weight, more preferably from 1% to 10% by weight, relative to the total weight of the composition.

The composition according to the invention comprises one or more non-polymeric thickening agents as other essential ingredient.

Preferably, the nonpolymeric thickening agents may be chosen from non-liquid fatty alcohols and non-liquid fatty esters at room temperature (25°C) and at atmospheric pressure (760 mmHg, i.e. 1.013×10⁵ Pa).

The term "non-liquid" means a solid compound or a compound that has a viscosity of greater than 2 Pa.s at a temperature of 25°C and at a shear rate of 1 s⁻¹.

More preferably the nonpolymeric thickening agents of the invention are solid at room temperature (25°C) and at atmospheric pressure (760 mmHg, i.e. 1.013×10⁵ Pa).

The non-liquid fatty alcohols are non oxyalkylelnatedand non glycerolated. They are particularly chosen from fatty alcohols, preferably monoalcohols, with C₁₀-C₃₀ linear alkyl chains, more preferably C₁₂-C₂₄ linear alkyl chains. The non-liquid fatty alcohols that may be used include stearyl alcohol, myristic alcohol, cetyl alcohol, and mixtures thereof. In some embodiments, mixtures of stearyl alcohol and of cetyl alcohol are used.

The non-liquid and preferably solid fatty esters are in particular selected from esters of linear or branched, more preferably linear, C₁₀-C₃₀, preferentially C₁₂-C₂₄ fatty alcohols and of linear or branched, more preferably linear, C₁₀-C₃₀, preferably C₁₂-C₂₄ fatty acids, such as cetyl palmitate, myristyl palmitate, stearyl palmitate, cetyl myristate. myristyl myristate and stearyl myristate, and mixtures thereof.

In one particular embodiment, the at least one non-polymeric thickening agent is chosen from non-liquid fatty alcohols.

Nonpolymeric thickening agent(s) may be used in an amount ranging from 0.5 to 20%, preferentially from 1 to 10% by weight, more preferably from 2 to 6% by weight relative to the total weight of the composition.

The composition comprises one or more amino silicones.

The term "amino silicone" is intended to mean any silicone comprising at least one primary, secondary or tertiary amine function or a quaternary ammonium group.

Preferably, the amino silicone(s) used in the cosmetic composition according to the present invention comprise(s) in its (their) structure at least 4 silicon atoms.

The amino silicones used in the composition according to the present invention can be chosen from:
(a) the compounds corresponding to formula (IX) below:

   (R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (IX)

   in which:
   T is a hydrogen atom or a phenyl, hydroxyl (-OH) or Ci-Cs alkyl group, and preferably methyl, or a Ci-Cs alkoxy, preferably methoxy,
   a denotes the number 0 or an integer from 1 to 3, and preferably 0,
   b denotes 0 or 1, and in particular 1,
   m and n are numbers such that the sum (n + m) can range especially from 1 to 2000 and in particular from 50 to 150, it being possible for n to denote a number from 0 to 1999 and in particular from 49 to 149, and for m to denote a number from 1 to 2000 and in particular from 1 to 10,
   R¹ is a monovalent group of formula -C_{q}H_{2q}L in which q is a number from 2 to 8 and L is an optionally quaternized amino group chosen from the following groups:

      -N(R²)-CH₂-CH₂-N(R²)₂,

      -N(R²)₂,

      -N⁺(R²)₃ Q⁻,

      -N⁺(R²)(H)₂ Q⁻,

      -N⁺(R²)₂HQ⁻,

      -N(R²)-CH₂-CH₂-N⁺(R²)(H)₂ Q⁻,
in which R² may denote a hydrogen atom, a phenyl, a benzyl or a saturated monovalent hydrocarbon-based group, for example a C₁-C₂₀ alkyl group, and Q⁻ represents a halide ion, for instance fluoride, chloride, bromide or iodide.

In particular, the amino silicones corresponding to the definition of formula (IX) are chosen from the compounds corresponding to formula (X) below: 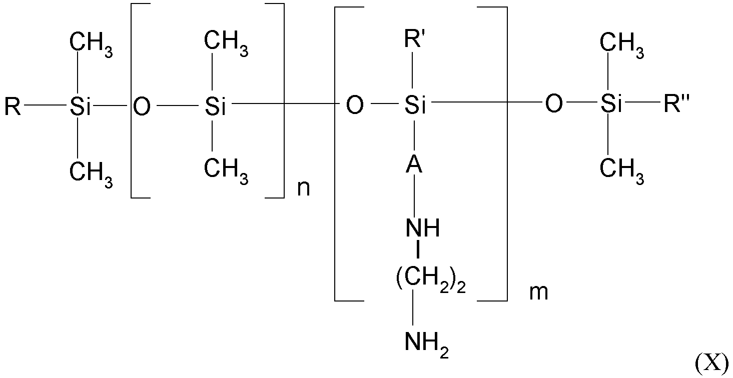 in which R, R' and R", which may be identical or different, denote a C₁-C₄ alkyl group, preferably CH₃; a C₁-C₄ alkoxy group, preferably methoxy; or OH; A represents a linear or branched, C₃-C₈ and preferably C₃-C₆ alkylene group; m and n are integers dependent on the molecular weight and whose sum is between 1 and 2000.

According to a first possibility, R, R' and R", which may be identical or different, represent a C₁-C₄ alkyl or hydroxyl group, A represents a C₃ alkylene group and m and n are such that the weight-average molecular mass of the compound is between 5000 and 500 000 approximately. Compounds of this type are referred to in the CTFA dictionary as "amodimethicones".

According to a second possibility, R, R' and R", which may be identical or different, each represent a C₁-C₄ alkoxy or hydroxyl group, at least one of the groups R or R" is an alkoxy group and A represents a C₃ alkylene group. The hydroxy/alkoxy mole ratio is preferably between 0.2/1 and 0.4/1 and advantageously equal to 0.3/1. Moreover, m and n are such that the weight-average molecular weight of the compound is between 2000 and 10⁶. More particularly, n is between 0 and 999 and m is between 1 and 1000, the sum of n and m being between 1 and 1000.

In this category of compounds, mention may be made, inter alia, of the product Belsil^{®} ADM 652 sold by the company Wacker.

According to a third possibility, R and R", which are different, each represent a C₁-C₄ alkoxy or hydroxyl group, at least one of the groups R or R" being an alkoxy group, R' representing a methyl group and A representing a C₃ alkylene group. The hydroxy/alkoxy mole ratio is preferably between 1/0.8 and 1/1.1 and advantageously is equal to 1/0.95. Moreover, m and n are such that the weight-average molecular weight of the compound is between 2000 and 200 000. More particularly, n is between 0 and 999 and m is between 1 and 1000, the sum of n and m being between 1 and 1000.

More particularly, mention may be made of the product Fluid WR^{®} 1300 sold by the company Wacker.

It should be noted that the molecular weight of these silicones is determined by gel permeation chromatography (ambient temperature, polystyrene standard, µ styragem columns, eluent THF, flow rate of 1 mm/minute, 200 µl of a solution containing 0.5% by weight of silicone in THF are injected, and detection is performed by refractometry and UV-metry).

A product corresponding to the definition of formula (IX) is in particular the polymer known in the CTFA dictionary as "trimethylsilyl amodimethicone", corresponding to formula (XI) below: 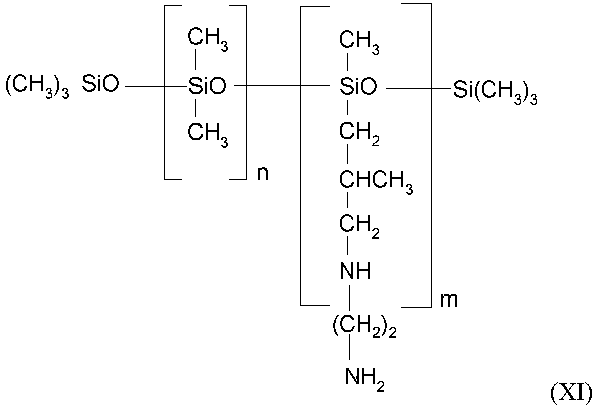 in which n and m have the meanings given above in accordance with formula (IX).

Such compounds are described, for example, in patent EP 95238. A compound of formula (IX) is sold, for example, under the name Q2-8220 by the company OSI.
(b) the compounds corresponding to formula (XII) below: 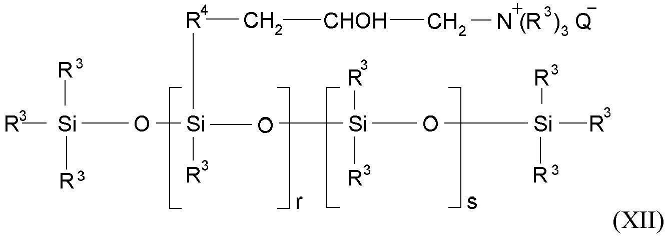 in which:
   R³ represents a C₁-C₁₈ monovalent hydrocarbon-based group, and in particular a C₁-C₁₈ alkyl or C₂-C₁₈ alkenyl group, for example methyl,
   R⁴ represents a divalent hydrocarbon-based group, especially a C₁-C₁₈ alkylene group or a divalent C₁-C₁₈, and for example Ci-Cs, alkylenoxy group,
   Q⁻ is a halide ion, in particular chloride;
   r represents a mean statistical value from 2 to 20 and in particular from 2 to 8;
   s represents a mean statistical value from 20 to 200 and in particular from 20 to 50.
   Such compounds are described more particularly in patent US 4 185 087.
   A compound falling within this category is the product sold by the company Union Carbide under the name Ucar Silicone ALE 56;
c) quaternary ammonium silicones especially of formula (XIII): 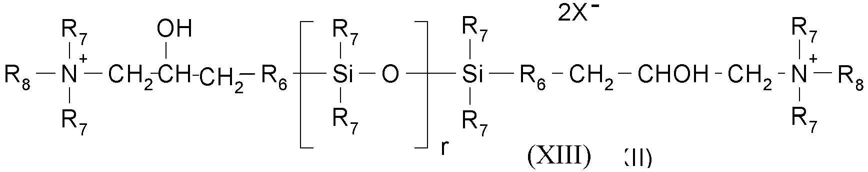 in which:
   R⁷, which may be identical or different, represent a monovalent hydrocarbon-based group containing from 1 to 18 carbon atoms, and in particular a C₁-C₁₈ alkyl group, a C₂-C₁₈ alkenyl group or a ring comprising 5 or 6 carbon atoms, for example methyl,
   R₆ represents a divalent hydrocarbon-based group, in particular a C₁-C₁₈ alkylene group or a divalent C₁-C₁₈, and for example C₁-C₈, alkylenoxy group linked to the Si via an SiC bond,
   R₈, which may be identical or different, each represent a hydrogen atom, a monovalent hydrocarbon-based group containing from 1 to 18 carbon atoms, and in particular a C₁-C₁₈ alkyl group, a C₂-C₁₈ alkenyl group or a group -R₅-NHCOR₇;
   X⁻ is an anion such as a halide ion, in particular chloride, or an organic acid salt (acetate, etc.);
   r represents a mean statistical value from 2 to 200 and in particular from 5 to 100.

These silicones are described, for example, in patent application EP-A 0 530 974.
d) the amino silicones of formula (XIV): 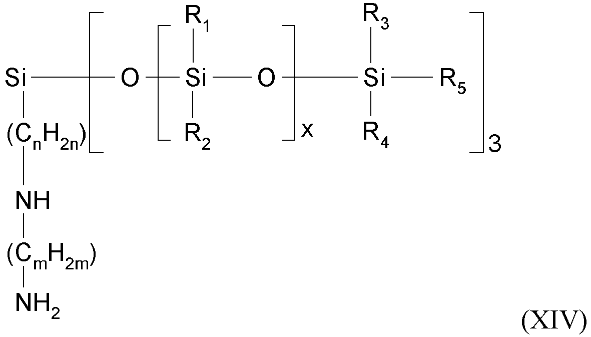 in which:
- R₁, R₂, R₃ and R₄, which may be identical or different, each denote a C₁-C₄ alkyl group or a phenyl group,
- R₅ denotes a C₁-C₄ alkyl group or a hydroxyl group,
- n is an integer ranging from 1 to 5,
- m is an integer ranging from 1 to 5, and
- x is chosen such that the amine number is between 0.01 and 1 meq/g.

When these compounds are used, one particularly advantageous embodiment involves their combined use with cationic and/or nonionic surfactants.

By way of example, use may be made of the product sold under the name Cationic Emulsion DC 939 by the company Dow Corning, which comprises, in addition to the amodimethicone, a cationic surfactant, namely trimethylcetylammonium chloride, and a nonionic surfactant of formula C₁₃H₂₇-(OC₂H₄)₁₂-OH, known under the CTFA name Trideceth-12.

Another commercial product that may be used according to the invention is the product sold under the name Dow Corning Q2 7224 by the company Dow Corning, comprising, in combination, the trimethylsilyl amodimethicone of formula (XI) described above, a nonionic surfactant of formula: C₈H₁₇-C₆H₄-(OCH₂CH₂)₄₀-OH, known under the CTFA name Octoxynol-40, a second nonionic surfactant of formula: C₁₂H₂₅-(OCH₂-CH₂)₆-OH, known under the CTFA name isolaureth-6, and propylene glycol.

Another commercial product that may be used according to the invention is the product sold under the name Wacker-Belsil ADM LOG 1, sold by the company Wacker, comprising, in microemulsion form, an amodimethicone of formula (X) in combination with Trideceth-5 and Trideceth-10.

Other amino silicones can be used in the context of the invention, such as the product referenced in the CTFA dictionary under the name Polysilicone-9.

Preferably, the amino silicone(s) used in the cosmetic composition according to the invention are chosen from amino silicones corresponding to formula (IX) and even more particularly from the amino silicones of formula (X) or (XI).

The amino silicone(s) may be present in an amount ranging from 0.01% to 10%, preferably from 0.1% to 5% by weight, and better still from 0.2% to 1% by weight, relative to the total weight of the composition.

The composition according to the invention may be aqueous or anhydrous.

The term "anhydrous" refers to a composition not containing any added water, i.e. a composition in which the water that may be present comes only from the water of crystallization or of adsorption of the starting materials. In any event, an anhydrous composition contains less than 5% by weight of water and preferably less than 1% by weight of water relative to the total weight of the composition.

Preferably, the composition according to the invention is aqueous and comprises at least 30% by weight of water and preferably at least 50% by weight of water relative to the total weight of the composition.

When the composition of the invention is aqueous, its pH is generally between 2 and 9 and in particular between 3 and 8. Preferably, the pH is less than 7. Even more preferentially, it ranges from 3 to 6, most preferably from 3.5 to 5.

It may be adjusted to the desired value by means of acidifying or basifying agents usually used in cosmetics for this type of application, or alternatively using standard buffer systems.

The compositions according to the invention may also comprise one or more additives chosen from cationic, anionic, nonionic, amphoteric or zwitterionic polymers, nonionic, anionic, amphoteric or zwitterionic surfactants, ceramides, pseudoceramides, vitamins and provitamins, including panthenol, water-soluble or liposoluble sunscreens, nacreous agents and opacifiers, sequestrants, solubilizers, antioxidants, antidandruff agents, anti-seborrhoeic agents, anti-hair-loss compounds and/or hair restorers, propenetrants, fragrances, peptizers and preserving agents, or any other additive conventionally used in the cosmetics field.

A person skilled in the art will take care to select the optional additives and the amounts thereof such that they do not harm the properties of the compositions of the present invention.

The compositions according to the invention may be more or less fluid and may have the appearance of an ointment, a milk, a lotion, a serum, a paste or a mousse or a cream.

Preferably, compositions of the inventions are creams.

The composition of the invention finds application especially in a wide variety of cosmetic treatments of the hair, including the scalp, especially for treating, protecting, caring for, cleansing and/or conditioning the hair.

The compositions according to the invention may be, in a non-limiting manner, used as hair conditioners.

In a preferred embodiment, the composition may be a leave-on or rinse-out composition, more preferentially a leave-on cream.

The present invention also relates to a process for conditioning the hair, which consists in applying to keratin materials an effective amount of a composition as described above.

This application may or may not be followed by a rinsing operation. More preferentially, the application is not be followed by a rinsing operation.

When the application of the composition is followed by rinsing, the leave-on time of the composition on the keratin materials ranges from a few seconds to 60 minutes, better still from 5 seconds to 30 minutes, even better still from 10 seconds to 10 minutes.

The present invention also relates to the use of the cosmetic composition according to the invention for conditioning the hair.

The invention also relates to the composition of the invention for use in the treatment of dandruff.

The example that follows, serves to illustrate the invention without, however, being limiting in nature.

### EXAMPLE

In the following example, all the amounts are shown as weight percentages of product relative to the total weight of the composition.

A composition (I) according to the invention, and a composition (II) comparative are prepared from the compounds indicated in Table 1 below.

**Table 1**

| Phase | Ingredients | Composition (I) % by weight | Composition (II) % by weight |
|---|---|---|---|
| A | Behenyl trimethyl ammonium chloride | 0.474 AM | 0.474 AM |
| A | Cetyl trimethyl ammonium chloride | 0.38 AM | 0.38 AM |
| A | Glycerol | 0.5 | 0.5 |
| A | 1-Hydroxy-4-methyl 6-trimethylpentyl 2-pyridone, monoethanolamine salt | 0.25 | - |
| B | Cetylstearylic alcohol (C16/C18 30/70) | 4.2 | 4.2 |
| B | Mineral oil | 1.5 | 1.5 |
| B | Isopropyl myristate | 3 | 3 |
| C | Polydimethylsiloxane with aminoethylaminopropyl groups, methoxy and/or hydroxy and alpha-omega silanols function | 0.2875 AM | 0.2875 AM |
| C | Preservative, fragrance | qs | qs |
| | Water | Qsp 100 | Qsp 100 |

| | | | |
|---|---|---|---|
| A.M.: Active material | | | |

On one hand, the ingredients of phase A are mixed together at 70°C with 50 % by weight of the total amount of water.

On the other hand, the ingredients of phase B are mixed together at 70°C. Phase B is then added to phase A at 70°C with stirring and then cooling. The remaining water is added.

The ingredients of phase C are added at room temperature with stirring.

### Stability test

| | Composition (I) (invention) After preparation | Composition (II) (comparative) After preparation |
|---|---|---|
| Aspect | Smooth cream, brilliant, of white color | Smooth cream, brilliant, of white color |
| pH | 4,2 | 4,3 |
| Viscosity (Rheomat, UD, at 25°C, M3, 30s) | 50 | 40 |

| | Composition (I) 2 months at room temperature | Composition (I) 2 months at 4°C | Composition (I) 2 months at 45°C |
|---|---|---|---|
| Aspect | Smooth cream, brilliant, of white color | Smooth cream, brilliant, of white color | Smooth cream, brilliant, of very slightly yellow color |
| pH | 4,4 | 4,15 | 4,15 |
| Viscosity (Rheomat, UD, at 25°C, M3, 30s) | 46 | 56 | 63 |

There is no significant evolution of the pH, the viscosity and the appearance of the composition according to the invention, after a storage of 2 months, whatever the temperature is (4°C, 25°C or 45°C).

Furthermore, there is no significant modification of the pH, of the viscosity and of the appearance of the composition, with or without the antidandruff.

### Evaluation results

Composition (I) is applied on natural cleansed brazilian hair.

The composition according to the invention has good absorption and leaves hair malleable. On wet hair after rinsing, the hair are easier to comb, more softness, more malleable, more coated, more hydrated and more lighter. On dry hair after drying, more lightness, more natural effect and more movement are obtained.

The durability of these cosmetic effects is good.

## Claims

1. Cosmetic composition comprising
∘ one or more cationic surfactants chosen from cetyltrimethylammonium, behenyltrimethylammonium, dipalmitoylethylhydroxyethylmethylammonium and distearoylethylhydroxyethylmethylammonium salts, and mixtures thereof,
∘ one or more antidandruff agents,
∘ one or more liquid non-siliceous fatty substances,
∘ one or more non-polymeric thickening agents, and
∘ one or more aminosilicones,
wherein:
the one or more antidandruff agents are chosen from 1-hydroxy-2-pyridone derivatives represented especially by formula (V): 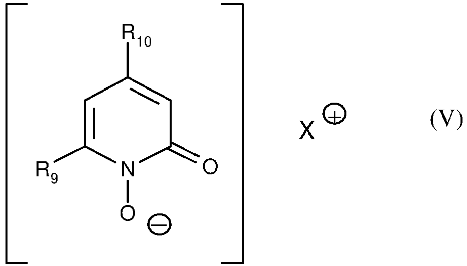
in which:
R₉ represents an alkyl group containing from 1 to 17 carbon atoms, an alkenyl group containing from 2 to 17 carbon atoms, a cycloalkyl group containing from 5 to 8 carbon atoms, a bicycloalkyl group containing from 7 to 9 carbon atoms; a cycloalkylalkyl group, an aryl group, an aralkyl group with an alkyl containing from 1 to 4 carbon atoms, an arylalkenyl group with an alkenyl containing from 2 to 4 carbon atoms, aryloxyalkyl or arylmercaptoalkyl with an alkyl containing from 1 to 4 carbon atoms, a furylalkenyl group with an alkenyl or a furyl containing from 2 to 4 carbon atoms, an alkoxy group containing from 1 to 4 carbon atoms, a nitro group, a cyano group or a halogen atom,
R₁₀ represents a hydrogen atom, a C₁-C₄ alkyl group, a C₂-C₄ alkenyl group, a halogen atom, a phenyl group or a benzyl group, and
X represents an organic base, an alkali metal or alkaline-earth metal ion or an ammonium ion;
and the one or more liquid non-siliceous fatty substances comprise isopropyl myristate.

2. Cosmetic composition according to claim 1, wherein the cationic surfactant(s) is or are chosen from behenyltrimethylammonium chloride, cetyltrimethylammonium chloride, dipalmitoylethylhydroxyethylammonium methosulfate and distearoylethylhydroxyethylmethylammonium methosulfate, and better still from behenyltrimethylammonium chloride, cetyltrimethylammonium chloride, and mixtures thereof.

3. Cosmetic composition according to any one of the preceding claims, wherein the cationic surfactant(s) is or are added in an amount ranging from 0.05% to 10% by weight, preferably from 0.1% to 5% by weight, more preferentially from 0.5% to 5% by weight and better still from 0.5% to 2% by weight relative to the total weight of the composition.

4. Cosmetic composition according to any one of the preceding claims, wherein the antidandruff agent(s) is or are chosen from (1-hydroxy-4-methyl-6-(2,4,4-trimethylpentyl)-2-pyridone salts.

5. Cosmetic composition according to any one of the preceding claims, wherein the antidandruff agent(s) is or are added in an amount preferentially ranging from 0.001% to 10% by weight, and more preferentially from 0.05% to 5% by weight, more preferably from 0.1 to 1% by weight, relative to the total weight of the composition.

6. Cosmetic composition according to any one of the preceding claims, wherein the liquid non-siliceous fatty substance(s) is (are) present in an amount ranging from 0.1% to 30% by weight, and more preferentially from 0.5% to 20% by weight, more preferably from 1% to 10% by weight relative to the total weight of the composition.

7. Cosmetic composition according to any one of the preceding claims, wherein the non-polymeric thickening agent(s) is (are) chosen from non-liquid fatty alcohols and non liquid fatty esters.

8. Cosmetic composition according to any one of the preceding claims, wherein the non-polymeric thickening agent(s) is (are) non-liquid fatty alcohol(s), preferably chosen from stearyl alcohol, myristic alcohol, cetyl alcohol, and mixtures thereof.

9. Cosmetic composition according to any one of the preceding claims, wherein the non-polymeric thickening agent(s) is (are) present in an amount ranging from 0.5 to 20%, preferentially from 1 to 10% by weight, more preferably from 2 to 6% by weight relative to the total weight of the composition.

10. The use of the composition as claimed in any one of the preceding claims, for conditioning the hair.

11. The cosmetic composition as claimed in any one of claims 1 to 9, for use in the treatment of dandruff.

12. The use of the composition as claimed in any one of the claims 1 to 9, as a leave-on cream.

## Patentansprüche

1. Kosmetische Zusammensetzung, umfassend
o ein oder mehrere kationische Tenside, ausgewählt aus Cetyltrimethylammonium-, Behenyltrimethylammonium-, Dipalmitoylethylhydroxyethylmethylammonium- und Distearoylethylhydroxyethylmethylammoniumsalzen und Mischungen davon,
o ein oder mehrere Antischuppenmittel,
o eine oder mehrere nicht siliciumhaltige Fettsubstanzen,
o ein oder mehrere nicht polymere Verdickungsmittel und
o ein oder mehrere Aminosilikone,
wobei:
das eine oder die mehreren Antischuppenmittel aus 1-Hydroxy-2-pyridon-Derivaten ausgewählt sind, besondere Formel (V) wiedergegeben werden: 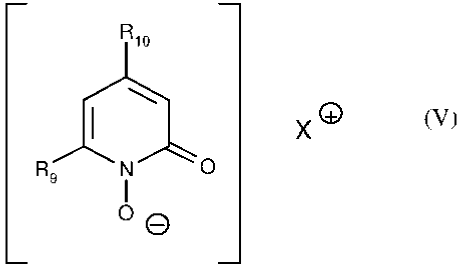
wobei:
R₉ für eine Alkylgruppe mit 1 bis 17 Kohlenstoffatomen, eine Alkenylgruppe mit 2 bis 17 Kohlenstoffatomen, eine Cycloalkylgruppe mit 5 bis 8 Kohlenstoffatomen, eine Bicycloalkylgruppe mit 7 bis 9 Kohlenstoffatomen, eine Cycloalkylalkylgruppe, eine Arylgruppe, eine Aralkylgruppe mit einem Alkyl mit 1 bis 4 Kohlenstoffatomen, eine Arylalkenylgruppe mit einem Alkenyl mit 2 bis 4 Kohlenstoffatomen, Aryloxyalkyl oder Arylmercaptoalkyl mit einem Alkyl mit 1 bis 4 Kohlenstoffatomen, eine Furylalkenylgruppe mit einem Alkenyl oder einem Furyl mit 2 bis 4 Kohlenstoffatomen, eine Alkoxygruppe mit 1 bis 4 Kohlenstoffatomen, eine Nitrogruppe, eine Cyanogruppe oder ein Halogenatom steht,
R₁₀ für ein Wasserstoffatom, eine C₁-C₄-Alkylgruppe, eine C₂-C₄-Alkenylgruppe, ein Halogenatom, eine Phenylgruppe oder eine Benzylgruppe steht und
X für eine organische Base, ein Alkalimetall- oder Erdalkalimetallion oder ein Ammoniumion steht;
und die eine oder die mehreren flüssigen nicht siliciumhaltigen Fettsubstanzen Isopropylmyristat umfassen.

2. Kosmetische Zusammensetzung nach Anspruch 1, wobei das eine kationische Tensid oder die mehreren kationischen Tenside aus Behenyltrimethylammoniumchlorid, Cetyltrimethylammoniumchlorid, Dipalmitoylethylhydroxyethylammoniummethosulfat und Distearoylethylhydroxyethylmethylammoniummethosulfat und noch besser aus Behenyltrimethylammoniumchlorid, Cetyltrimethylammoniumchlorid und Mischungen davon ausgewählt ist bzw. sind.

3. Kosmetische Zusammensetzung nach einem der vorhergehenden Ansprüche, wobei das kationische Tensid bzw. die kationischen Tenside in einer Menge im Bereich von 0,05 bis 10 Gew.-%, vorzugsweise von 0,1 bis 5 Gew.- %, weiter bevorzugt von 0,5 bis 5 Gew.-% und noch besser von 0,5 bis 2 Gew.-%, bezogen auf das Gesamtgewicht der Zusammensetzung, zugegeben wird bzw. werden.

4. Kosmetische Zusammensetzung nach einem der vorhergehenden Ansprüche, wobei das Antischuppenmittel bzw. die Antischuppenmittel aus (1-Hydroxy-4-methyl-6-(2,4,4-trimethylpentyl)-2-pyridon-Salzen ausgewählt ist bzw. sind.

5. Kosmetische Zusammensetzung nach einem der vorhergehenden Ansprüche, wobei das Antischuppenmittel bzw. die Antischuppenmittel bevorzugt in einer Menge im Bereich von 0,001 bis 10 Gew.-% und weiter bevorzugt von 0,05 bis 5 Gew.-%, weiter bevorzugt von 0,1 bis 1 Gew.- %, bezogen auf das Gesamtgewicht der Zusammensetzung, zugegeben wird bzw. werden.

6. Kosmetische Zusammensetzung nach einem der vorhergehenden Ansprüche, wobei die flüssige nicht siliciumhaltige Fettsubstanz bzw. die flüssigen nicht siliciumhaltigen Fettsubstanzen in einer Menge im Bereich von 0,1 bis 30 Gew.-% und weiter bevorzugt von 0,5 bis 20 Gew.-%, weiter bevorzugt von 1 bis 10 Gew.-%, bezogen auf das Gesamtgewicht der Zusammensetzung, vorliegt bzw. vorliegen.

7. Kosmetische Zusammensetzung nach einem der vorhergehenden Ansprüche, wobei das nicht polymere Verdickungsmittel bzw. die nicht polymeren Verdickungsmittel aus nicht flüssigen Fettalkoholen und nicht flüssigen Fettestern ausgewählt ist bzw. sind.

8. Kosmetische Zusammensetzung nach einem der vorhergehenden Ansprüche, wobei es sich bei dem nicht polymeren Verdickungsmittel bzw. den nicht polymeren Verdickungsmitteln um einen oder mehrere nicht flüssige Fettalkohole, vorzugsweise ausgewählt aus Stearylalkohol, Myristylalkohol, Cetylalkohol und Mischungen davon, handelt.

9. Kosmetische Zusammensetzung nach einem der vorhergehenden Ansprüche, wobei das nicht polymere Verdickungsmittel bzw. die nicht polymeren Verdickungsmittel in einer Menge im Bereich von 0,5 bis 20 Gew.-%, vorzugsweise von 1 bis 10 Gew.-%, weiter bevorzugt von 2 bis 6 Gew.-%, bezogen auf das Gesamtgewicht der Zusammensetzung, vorliegt bzw. vorliegen.

10. Verwendung der Zusammensetzung nach einem der vorhergehenden Ansprüche zum Konditionieren des Haars.

11. Kosmetische Zusammensetzung nach einem der Ansprüche 1 bis 9 zur Anwendung bei der Behandlung von Schuppen.

12. Verwendung der Zusammensetzung nach einem der Ansprüche 1 bis 9 als Leave-on-Creme.

## Revendications

1. Composition cosmétique comprenant
o un ou plusieurs tensioactifs cationiques choisis parmi les sels de cétyltriméthylammonium, de béhényltriméthylammonium, de dipalmitoyléthylhydroxyéthylméthylammonium et de distéaroyléthylhydroxyéthylméthylammonium et leurs mélanges,
o un ou plusieurs agents antipelliculaires,
o une ou plusieurs substances grasses liquides non siliceuses,
o un ou plusieurs agents épaississants non polymériques, et
o une ou plusieurs aminosilicones,
dans laquelle :
l'agent ou les agents antipelliculaires sont choisis parmi les dérivés de 1-hydroxy-2-pyridone représentés notamment par la formule (V) : 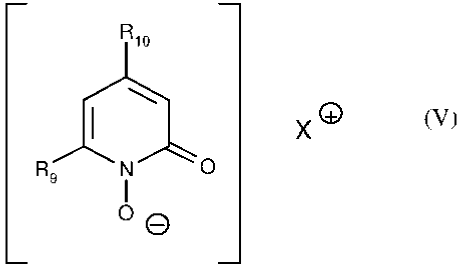
dans laquelle :
R₉ représente un groupe alkyle contenant de 1 à 17 atomes de carbone, un groupe alcényle contenant de 2 à 17 atomes de carbone, un groupe cycloalkyle contenant de 5 à 8 atomes de carbone, un groupe bicycloalkyle contenant de 7 à 9 atomes de carbone ; un groupe cycloalkylalkyle, un groupe aryle, un groupe aralkyle avec un alkyle contenant de 1 à 4 atomes de carbone, un groupe arylalcényle avec un alcényle contenant de 2 à 4 atomes de carbone, un aryloxyalkyle ou arylmercaptoalkyle avec un alkyle contenant de 1 à 4 atomes de carbone, un groupe furylalcényle avec un alcényle ou un furyle contenant de 2 à 4 atomes de carbone, un groupe alcoxy contenant de 1 à 4 atomes de carbone, un groupe nitro, un groupe cyano ou un atome d'halogène,
R₁₀ représente un atome d'hydrogène, un groupe alkyle en C₁-C₄, un groupe alcényle en C₂-C₄, un atome d'halogène, un groupe phényle ou un groupe benzyle, et
X représente une base organique, un ion de métal alcalin ou de métal alcalino-terreux ou un ion ammonium ;
et la ou les substances grasses non siliceuses liquides comprennent du myristate d'isopropyle.

2. Composition cosmétique selon la revendication 1, dans laquelle le ou les tensioactifs cationiques est/sont choisi(s) parmi le chlorure de béhényltriméthylammonium, le chlorure de cétyltriméthylammonium, le méthosulfate de dipalmitoyléthylhydroxyéthylammonium et le méthosulfate de distéaroyléthylhydroxyéthylméthylammonium, et mieux encore parmi le chlorure de béhényltriméthylammonium, le chlorure de cétyltriméthylammonium et leurs mélanges.

3. Composition cosmétique selon l'une quelconque des revendications précédentes, dans laquelle le ou les tensioactifs cationiques est/sont ajouté(s) en une quantité dans la plage de 0,05 % à 10 % en poids, de préférence de 0,1 % à 5 % en poids, plus préférentiellement de 0,5 % à 5 % en poids et mieux encore de 0,5 % à 2 % en poids par rapport au poids total de la composition.

4. Composition cosmétique selon l'une quelconque des revendications précédentes, dans laquelle l'agent ou les agents antipelliculaires est/sont choisi(s) parmi les sels de (1-hydroxy-4-méthyl-6-(2,4,4-triméthylpentyl)-2-pyridone.

5. Composition cosmétique selon l'une quelconque des revendications précédentes, dans laquelle l'agent ou les agents antipelliculaires est/sont ajouté(s) en une quantité préférentiellement dans la plage de 0,001 % à 10 % en poids, et de préférence de 0,05 % à 5 % en poids, plus préférablement de 0,1 à 1 % en poids, par rapport au poids total de la composition.

6. Composition cosmétique selon l'une quelconque des revendications précédentes, dans laquelle la ou les substance(s) grasse(s) liquide(s) non siliceuse(s) est/sont présente(s) en une quantité dans la plage de 0,1 % à 30 % en poids, et plus préférentiellement de 0,5 % à 20 % en poids, plus préférablement de 1 % à 10 % en poids par rapport au poids total de la composition.

7. Composition cosmétique selon l'une quelconque des revendications précédentes, dans laquelle l'agent ou les agents épaississant(s) non polymérique(s) est/sont choisi (s) parmi des alcools gras non liquides et des esters gras non liquides.

8. Composition cosmétique selon l'une quelconque des revendications précédentes, dans laquelle l'agent ou les agents épaississant(s) non polymérique(s) est/sont un ou plusieurs alcools gras non liquides, de préférence choisis parmi l'alcool stéarylique, l'alcool myristique, l'alcool cétylique et des mélanges.

9. Composition cosmétique selon l'une quelconque des revendications précédentes, dans laquelle l'agent ou les agents épaississant(s) non polymérique(s) est/sont présent(s) en une quantité dans la plage de 0,5 à 20 %, de préférence de 1 à 10 % en poids, plus préférablement de 2 à 6 % en poids par rapport au poids total de la composition.

10. Utilisation de la composition selon l'une quelconque des revendications précédentes, pour le conditionnement des cheveux.

11. Composition cosmétique selon l'une quelconque des revendications 1 à 9, pour une utilisation dans le traitement des pellicules.

12. Utilisation de la composition selon l'une quelconque des revendications 1 à 9, comme crème sans rinçage.
